(19) Europäisches Patentamt
European Patent Office
Office européen des brevets

(11) Veröffentlichungsnummer: **0 351 746 B1**

(12) # EUROPÄISCHE PATENTSCHRIFT

(45) Veröffentlichungstag der Patentschrift: **29.12.93**

(51) Int. Cl.⁵: **C07D 317/24**, C07D 405/12, C07D 405/14, C07D 409/12, C07D 409/14, C07D 417/12, C07D 417/14, C09K 19/58, G02F 1/33

(21) Anmeldenummer: **89113045.2**

(22) Anmeldetag: **15.07.89**

(54) **Optisch aktive, in 4-Stellung einen mesogenen Rest tragende 1,3-Dioxolan-Derivate, ein Verfahren zu ihrer Herstellung und ihre Verwendung als Dotierstoffe in Flüssigkristallmischungen.**

(30) Priorität: **22.07.88 DE 3824902**

(43) Veröffentlichungstag der Anmeldung: **24.01.90 Patentblatt 90/04**

(45) Bekanntmachung des Hinweises auf die Patenterteilung: **29.12.93 Patentblatt 93/52**

(84) Benannte Vertragsstaaten: **AT BE CH DE FR GB IT LI NL SE**

(56) Entgegenhaltungen:
**EP-A- 0 234 437**
**EP-A- 0 288 813**
**DE-A- 3 604 899**
**DE-A- 3 739 588**
**US-A- 4 424 372**

(73) Patentinhaber: **HOECHST AKTIENGESELLSCHAFT**
**D-65926 Frankturt(DE)**

(72) Erfinder: **Scherowsky, Günter, Prof.**
**Winklerstrasse 18B**
**D-1000 Berlin 33(DE)**
Erfinder: **Gay, Jürgen**
**Bismarckstrasse 51A**
**D-1000 Berlin 33(DE)**
Erfinder: **Wingen, Rainer, Dr.**
**Rotkäppchenweg 10**
**D-6234 Hattersheim am Main(DE)**
Erfinder: **Dübal, Hans-Rolf, Dr.**
**Heuhohlweg 6**
**D-6240 Königstein/Taunus(DE)**
Erfinder: **Escher, Claus, Dr.**
**Amselweg 3**
**D-6109 Mühltal(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

**CHEMICAL ABSTRACTS, Band 96, Nr. 26, 28. Juni 1982, Columbus, Ohio, USA SCHLEIER, GISBERT et al. "Chiral liquid crystal polymers. 1. Synthesis and properties of glycidyl ethers containing anisotropic groups" Seite 1, Spalte 2, Zusammen- fassung-Nr. 218 245n**

**CHEMICAL ABSTRACTS, Band 107, Nr. 12, 21. September 1987, Columbus, Ohio, USA FUJIMURA, HIROSHI et al. "Liquid crystal compositions for display devices" Seite 662, Spalte 2, Zusammenfassung-Nr. 106 508q**

Erfinder: **Hemmerling, Wolfgang, Dr.**
**Billtalstrasse 32**
**D-6231 Sulzbach (Taunus)(DE)**
Erfinder: **Inoguchi, Yoshio, Dr.**
**4-12-201, Fujicho 4-chome**
**Hohya-shi Tokyo(JP)**
Erfinder: **Müller, Ingrid, Dr.**
**Am Pfingstbrunnen 1**
**D-6238 Hofheim am Taunus(DE)**
Erfinder: **Ohlendorf, Dieter, Dr.**
**Am Kühlen Grund 4**
**D-6237 Liederbach(DE)**

## Beschreibung

Flüssigkristalle haben insbesondere im letzten Jahrzehnt Eingang in verschiedene technische Gebiete gefunden, in denen elektrooptische und Anzeigevorrichtungs-Eigenschaften gefragt sind (z.B. in Uhren-, Taschenrechner- und Schreibmaschinenanzeigen). Diese Anzeigevorrichtungen beruhen auf den dielektrischen Ausrichtungseffekten in den nematischen, cholesterischen und smektischen Phasen der flüssigkristallinen Verbindungen, wobei - verursacht durch die dielektrische Anisotropie - die molekulare Längsachse der Verbindungen eine bevorzugte Ausrichtung in einem angelegten elektrischen Feld einnimmt. Die üblichen Schaltzeiten bei diesen Anzeigevorrichtungen sind für viele andere potentielle Anwendungsgebiete von Flüssigkristallen, die an sich für die Technik wegen ihrer einzigartigen Eigenschaften sehr vielversprechende chemische Verbindungen sind, eher zu lang. Dieser Nachteil macht sich insbesondere dann bemerkbar, wenn eine große Anzahl von Bildpunkten angesteuert werden muß, wodurch die Herstellungskosten von Geräten, die größere Flächen enthalten, z.B. Videogeräte, Oszillographen oder Fernseh-, Radar-, EDV- oder Schreibautomaten-Bildschirme, zu hoch werden.

Neben den nematischen und cholesterischen Flüssigkristallen haben seit einigen wenigen Jahren in zunehmendem Maß auch optisch aktive, smektische Flüssigkristall-Phasen an Bedeutung gewonnen.

Clark und Lagerwall konnten zeigen, daß die Verwendung ferroelektrischer Flüssigkristallsysteme in sehr dünnen Zellen zu optoelektrischen Schalt- oder Anzeigeelementen führt, die im Vergleich mit den herkömmlichen TN ("twisted nematic")-Zellen um bis zu einem Faktor 1000 schnellere Schaltzeigen haben (vgl. z.B. Lagerwall et al. "Ferroelectric Liquid Crystals for Displays", SID Symposium, October Meeting 1985, San Diego, Ca., USA). Aufgrund dieser und anderer günstiger Eigenschaften, z.B. der bistabilen Schaltmöglichkeit und des nahezu blickwinkelunabhängigen Kontrastes, sind ferroelektrische Flüssigkristalle grundsätzlich für die obengenannten Anwendungsgebiete, z.B. über eine Matrixansteuerung, gut geeignet. Man benötigt dazu entweder Verbindungen, die geneigt-smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen chirale, geneigt-smektische Phasen induzieren. Die gewünschte Phase soll dabei über eine möglichst großen Temperaturbereich stabil sein.

Zur Erzielung eines guten Kontrastverhältnisses in elektrooptischen Bauelementen ist eine einheitliche planare Orientierung der Flüssigkristalle nötig. Eine gute Orientierung in der $S_A^*$ - und $S_C^*$ -Phase läßt sich erreichen, wenn die Phasenfolge der Flüssigkristallmischung mit abnehmender Temperatur lautet:

Isotrop $\rightarrow N^* \rightarrow S_A^* \rightarrow S_C^*$ .

Voraussetzung ist, daß der pitch (Ganghöhe der Helix) in der N*-Phase sehr groß (> 10 $\mu$m) oder noch besser völlig kompensiert ist. (T. Matsumoto et al., p. 468-470, Proc. of the 6th Int. Display Research Conf., Japan Display, Sept 30 - Oct. 2, 1986, Tokyo, Japan; M. Murakami et al., ibid. p. 344 - p. 347). Dies erreicht man, indem man zu der chiralen Flüssigkristallmischung, die in der N*-Phase z.B. eine linksdrehende Helix aufweist, einen weiteren optisch aktiven Dotierstoff, der eine rechtsdrehende Helix induziert, in solchen Mengen hinzugibt, daß die Helix gerade oder wenigstens annähernd (Ganghöhe > 10 $\mu$m) kompensiert wird.

Aus dem Stand der Technik sind zwar schon Verbindungen bekannt, die einige der vorstehend beschriebenen Eigenschaften aufweisen, da aber die Herstellung praxisgerechter Flüssigkristallmischungen - je nach Art und Anzahl der Komponenten - ein in hohem Maße komplexer Vorgang ist, wird eine breite Auswahl an Dotierstoffen benötigt. Insbesondere werden solche Verbindungen gesucht, die bei geringer Zusatzmenge ein hohes Verdrillungsvermögen aufweisen und die vorstehend beschriebene Kompensation bewirken, ohne dabei die übrigen geforderten und deshalb "komponierten" Eigenschaften der Mischungen (z. B. eine hohe spontane Polarisation $P_s$) negativ zu beeinflussen. Aufgabe der vorliegenden Erfindung ist es deshalb, neue Verbindungen mit diesen Eigenschaften zu synthetisieren, die mit unterschiedlichsten Flüssigkristallmischungen verträglich sind.

Ein Gegenstand der Erfindung sind optisch aktive, in 4-Stellung einen mesogenen Rest tragende 1,3-Dioxolan-Derivate der allgemeinen Formel (I)

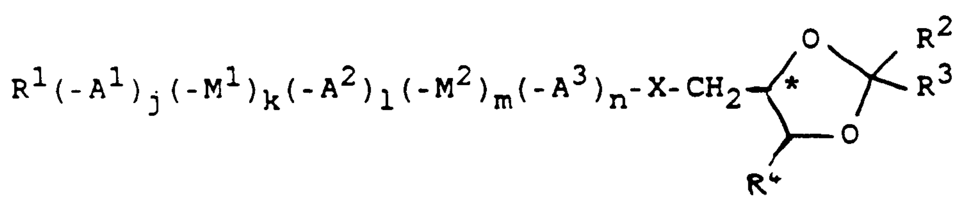

(I)

in der die Symbole folgende Bedeutung haben:

$R^1$

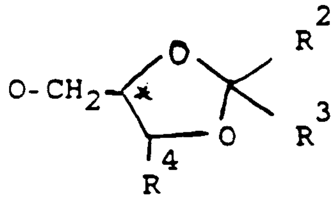

oder

ein geradkettiger oder verzweigter Alkylrest mit 1 bis 16 C-Atomen oder ein geradkettiger oder verzweigter Alkenylrest mit 3 bis 16 C-Atomen, wobei diese Reste selbst asymmetrische C-Atome enthalten können, wobei eine oder mehrere nicht benachbarte $-CH_2$-Gruppen durch -O-, -S-, -CO-, -O-CO- und/oder -CO-O- und wobei ein oder mehrere H durch F, Cl, Br oder CN ersetzt sein können

$R^2$ und $R^3$ jeweils H oder ein Alkylrest mit 1 bis 10 C-Atomen, wobei ein oder mehrere H des Alkylrestes durch F ersetzt sein können, oder $R^2$ und $R^3$ bilden zusammen mit dem C-(2)-Atom des Dioxolanrings einen Cyclopentan-, Cyclohexan- oder Cycloheptanring oder eine Ketogruppe

$R^4$ H oder ein Alkylrest mit 1 bis 10 oder ein Alkenylrest mit 2 bis 10 C-Atomen

j und l null, 1 oder 2

k und m null oder 1

n null, 1 oder 2 mit folgender Maßgabe: wenn j und/oder l = null sind, ist k = null; wenn n = null ist, ist m = null; die Summe j + l + n ist minimal 1 und maximal 3,

$-A^1$ und $-A^2$

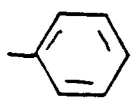 , 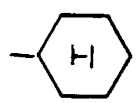 , 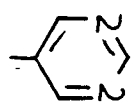 , 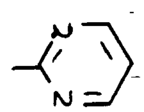 ,

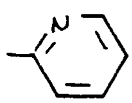 , 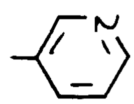 , 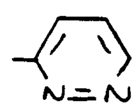 , 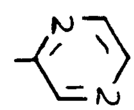 ,

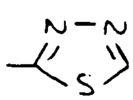 , 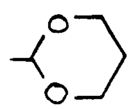 , 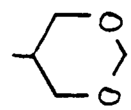 , 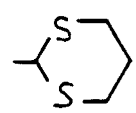 ,

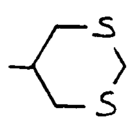 , 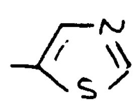 , 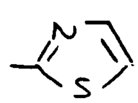 ,

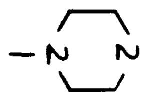 , 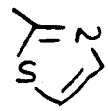 , 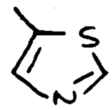

$-A^3$

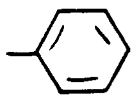 , 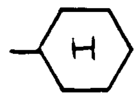 , 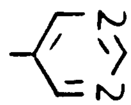 , 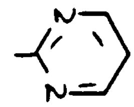 ,

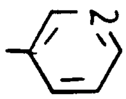 , 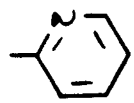 , 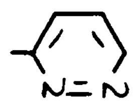

$-M^1$ und $-M^2$
: -CO-O, -O-CO, $-CH_2CH_2$, -CH=CH, $-CH_2O$, $-OCH_2$, -C≡C

X
: O, S oder O-CO-O.

Die obigen Formeln sind dabei so zu verstehen, daß die nächste Gruppe am äußersten rechten Molekülteil anschließt.

In einer bevorzugten Ausführungsform haben die Symbole in der allgemeinen Formel (I) die folgende Bedeutung:

- $R^1$ — ein geradkettiger oder verzweigter Alkyl- oder Alkenylrest mit 4 bis 14 C-Atomen, der ein asymmetrisches C-Atom enthalten kann, oder wobei eine $-CH_2$-Gruppe durch -O-, -S- oder -O-CO- ersetzt sein kann, oder wobei ein oder mehrere H durch F ersetzt sein können,
- $R^2$,$R^3$,$R^4$ — H oder ein Alkylrest mit 1 bis 5 C-Atomen oder $R^2$,$R^3$ zusammen mit dem C(2)-Atom des Dioxolanrings einen Cyclopentan- oder Cyclohexanring oder eine Ketogruppe
- j und l — null oder 1,
- k,m,n — null oder 1
- $-M^1$,$M^2$ — -CO-O, -O-CO
- X — O oder O-CO-O.

In einer weiteren bevorzugten Ausführungsform haben die 1,3-Dioxolan-Derivate die allgemeine Formel (IV)

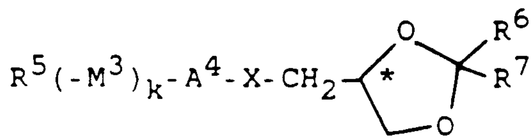

worin bedeuten:

- $R^6$, $R^7$ — Methyl oder zusammen mit den C(2)-Atom des Dioxolanrings einen Cyclohexanring,
- $R^5$ — einen geradkettigen oder verzweigten Alkyl- oder Alkenylrest mit 6 bis 12 C-Atomen, der ein asymmetrisches C-Atom enthalten kann,
- $-M^3$ — -O, -S oder -O-CO,
- $-A^4$

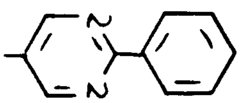 , 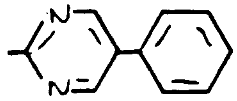 , 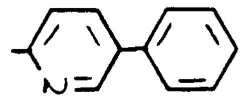 ,

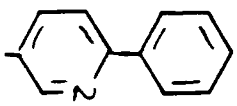 , 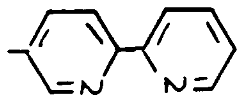 , 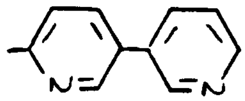 ,

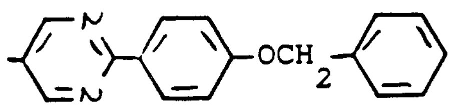 , 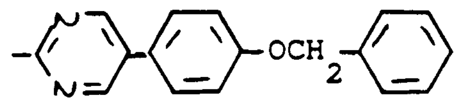 ,

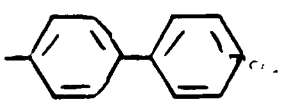 , 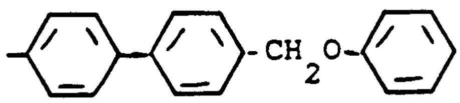 ,

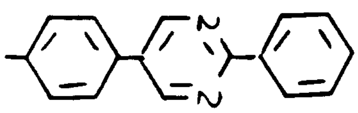 , 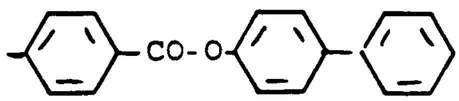 ,

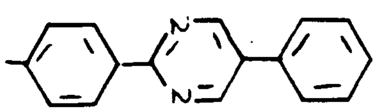 oder 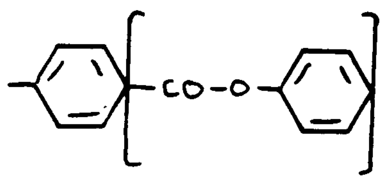 1 oder 2

Zu den neuen Verbindungen der allgemeinen Formel I, insbesondere (IV), gehören bevorzugt die in den Beispielen namentlich genannten Verbindungen.

Zur Herstellung der Verbindungen der allgemeinen Formel (I) werden mesogene Phenole, Thiophenole oder Kohlensäuremonoester der allgemeinen Formel (II)

$$R^1(-A^1)_j(-M^1)_k(-A^2)_l(-M^2)_m(-A^3)_n\text{-X-H} \qquad \text{(II)}$$

mit geeigneten Derivaten des 1,3-Dioxolans (III), wobei Y für eine typische Abgangsgruppe steht,

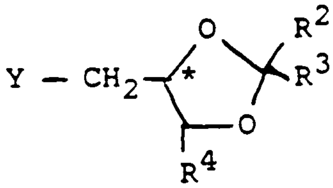 (III)

umgesetzt.

So können Verbindungen mit X = 0 in (II) und Y = OH in (III) in Gegenwart von Azodicarbonsäurediester und Triphenylphosphin zu (I) umgesetzt werden; dies in Anwendung einer Methode, die dem Fachmann als Mitsunobo-Reaktion bekannt ist (siehe z. B. J. Chem. Soc. Perkin Trans 1975, 461). Es können aber

auch sich von (II) ableitende Alkali-oder Erdalkalisalze mit (III) umgesetzt werden, in denen Y für eine typische Abgangsgruppe wie Methylsulfonyl, Trifluormethylsulfonyl, 4-Toluolsulfonyl oder eine andere dem Fachmann bekannte Abgangsgruppe (aus Standardliteratur über nukleophile Substitutionsreaktionen) steht.

Methoden zur Herstellung von Verbindungen des Typs (II) sind dem Fachmann bekannt (z. B. Zaschke et al., Flüssige Kristalle in Tabellen, Bände I und II, ...), die Kohlensäuremonoester vom Typ (II) werden beispielsweise aus den entsprechenden Phenolen und Phosgen in Gegenwart einer organischen Base hergestellt.

Die Verbindungen (III) sind im allgemeinen im Handel zu beziehen oder können nach Literaturstellen wie Tetrahedron 42, 447 (1986) hergestellt werden. Die Reinigung der Verbindungen (I) kann in der Regel durch Chromatographie und/oder Krisatllisation erfolgen.

Die eine weitere Lösung der gestellten Aufgabe darstellenden, insbesondere ferroelektrischen Flüssigkristallmischungen bilden Flüssigkristall-Phasen und enthalten mindestens eine optisch aktive Verbindung der allgemeinen Formel (I).

Unter dem Begriff "Flüssigkristallphase" sind nematische, cholesterische, orthogonal smektische oder geneigt ("tilted")-smektische, insbesondere $S_c^*$ -Phasen zu verstehen. Die Flüssigkristallmischungen bestehen aus 2 bis 20, vorzugsweise 2 bis 15 Komponenten, darunter mindestens eine der erfindungsgemäß beanspruchten chiralen Verbindungen.

Die anderen Bestandteile werden vorzugsweise ausgewählt aus den bekannten Verbindungen mit nematischen, cholesterischen und/oder smektischen Phasen; dazu gehören beispielsweise Schiffsche Basen, Biphenyle, Terphenyle, Phenylcyclohexane, Cyclohexylbiphenyle, N, S oder O-haltige Heterocyclen, z.B. Pyrimidine, Zimtsäureester, Cholesterinester oder verschieden überbrückte, terminalpolar mehrkernige Ester von p-Alkylbenzoesäuren. Im allgemeinen liegen die im Handel erhältlichen Flüssigkristallmischungen bereits vor der Zugabe der optisch aktiven Verbindung(en) als Gemische verschiedenster Komponenten vor, von denen mindestens eine mesogen ist, d.h. als Verbindung, in derivatisierter Form oder im Gemisch mit bestimmten Cokomponenten eine Flüssigkristallphase zeigt, die mindestens eine enantiotrope (Klärtemperatur > Schmelztemperatur) oder monotrope (Klärtemperatur < Schmelztemperatur) Mesophasenbildung erwarten läßt.

Insbesondere enthält die Flüssigkristall-Mischung neben mindestens einer der erfindungsgemäß beanspruchten optisch aktiven Verbindungen eine Esterverbindung mit $S_c$-Phase, z.B. einen Alkoxybenzoesäurephenylester, oder eine biaromatische Verbindung mit einem stickstoffhaltigen Heterocyclus, z.B. ein Alkylpyrimidinyl-alkoxy-benzol.

Von der oder den erfindungsgemäßen Verbindung(en) enthalten die Flüssigkristallmischungen im allgemeinen 0,05 bis 70 Gew.-%, insbesondere 0,1 bis 50 Gew.-%.

Die erfindungsgemäßen Verbindungen sind als Dotierstoffe für - insbesondere ferroelektrische - Flüssigkristallmischungen mit nematischen, cholesterischen und/oder smektischen Phasen geeignet. Sie weisen beispielsweise in nematischen bzw. cholesterischen Phasen ein hohes Verdrillungsvermögen auf, wobei bereits geringe Zugabemengen ausreichen, die eingangs beschriebene Helixkompensation durchzuführen, ohne dabei andere bereits vorhandene Eigenschaften der dotierten Mischung wesentlich negativ zu beeinflussen. Eine solche hochspezifische Wirksamkeit ist bei der Entwicklung praxisgerechter Mischungen außerordentlich vorteilhaft.

Insbesondere bewirken die erfindungsgemäßen Verbindungen in $S_c$-Phasen nur eine geringfügige spontane Polarisation $P_s$, so daß sie zwar die Ganghöhe einer Mischung stark, die Polarisation aber kaum beeinflussen. Die Verbindungen eignen sich daher bevorzugt zum Einsatz in ferroelektrischen Mischungen unabhängig vom Vorzeichen der dort vorhandenen spontanen Polarisation [z. B. R. B. Meyer et al., J. Phys. (Paris) Lett. 36, L-69 (1975)]. Auch in der $S_c$-Phase induzieren die erfindungsgemäßen Verbindungen eine Helix, so daß dieser Effekt benutzt werden kann, eine Verdrillung in der $S_c^*$ -Phase zu kompensieren oder auf einen bestimmten Wert einzustellen, was für den praktischen Einsatz vorteilhaft ist [z. B. T. Tsuchiga et al., Jpn. J. Appl. Phys. 25, L-27 (1986)].

Das hohe Verdrillungsvermögen der erfindungsgemäßen Verbindungen führt bereits in nematischen Phasen bei den eher "klassischen" Displaytechnologien zu vorteilhaften Einsatzmöglichkeiten. Hierbei steht jedoch häufig nicht die Kompensation, sondern die Erzielung einer Verdrillung durch eine möglichst geringe Zugabemenge an chiralem Dotierstoff im Vordergrund. Dies gilt sowohl für die z. Z. (noch) marktbeherrschende TN ("twisted-nematic")-Technologie [M. Schadt et al., Appl. Phys. Lett 18, 127 (1971)] als auch für das sogenannte White-Taylor-Display [D. L. White et al., J. Appl. Phys. 45, 4718 (1974)] oder das SBE/STN ("super-birefringence-effect"/"super-twisted-nematic")-Display [T. J. Scheffer et al., Appl. Phys. Let. 45, 1021 (1984)] und seine verschiedenen Modifikationen wie das OMI ("optical mode interference")Display [M. Schadt et al., Appl. Phys. Lett. 50, 236 (1987)].

**Beispiel 1**

(S)-4-(5-Octyl-pyrimidin-2-yl)phenyl-(2,2-dimethyl-1,3-dioxolan-4-yl)methyl-ether

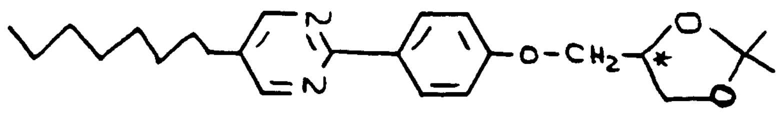

Zur Lösung von 1,38 g Triphenylphosphin in 30 ml Tetrahydrofuran werden 0,83 ml Azodicarbonsäurediethylester bei 0 °C gegeben. Nach 15 min wird mit 1,5 g 4-(5-Octylpyrimidin-2-yl)phenolund 0,7 g (S)-2,2-Dimethyl-1,3-dioxolan-4-methanol versetzt und das Gemisch wird während 2 Tagen bei 25 °C gehalten. Das Lösemittel wird abdestilliert und der Rückstand chromatographisch aufgetrennt ($SiO_2$, $CH_2Cl_2$/Essigsäureethylester 95/5). Nach dem Umkristallisieren aus n-Hexan werden 0,95 g des gewünschten Produkts erhalten, das einen Schmelzpunkt von 114,2 °C hat.
$[\alpha]_D^{22}$ : + 6,6 (c = 5, $CHCl_3$)
Analog werden erhalten:

**Beispiel 2**

(S)-4-(2-Octyloxy-pyrimidin-5-yl)phenyl-(2,2-dimethyl-1,3-dioxolan-4-yl)methyl-ether

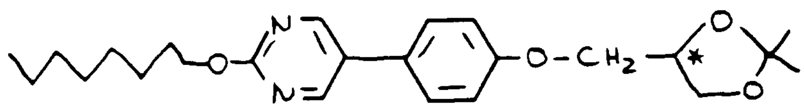

Schmelzpunkt: 90,2 °C $[\alpha]_D^{22}$ : + 4,1 (c = 5, $CHCl_3$)

**Beispiel 3**

(S)-4-(5-Octylthio-pyrimidin-5-yl)phenyl-(2,2-dimethyl-1,3-dioxolan-4-yl)methyl-ether

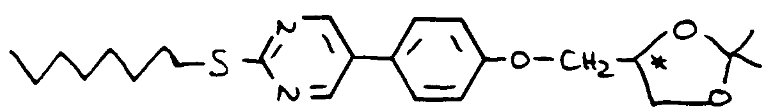

Schmelzpunkt: 86,8 °C $[\alpha]_D^{22}$ : + 4,0 (c = 5, $CHCl_3$)

**Beispiel 4**

(S)-4-(2-Octyl-pyrimidin-5-yl)phenyl-(2,2-dimethyl-1,3-dioxolan-4-yl)methyl-ether

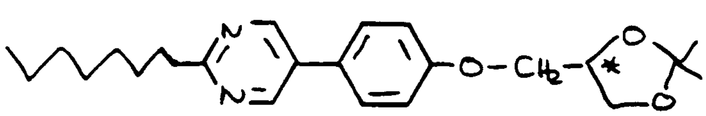

Schmelzpunkt: 95,6 °C $[\alpha]_D^{22}$ : + 4,4 (c = 5, $CHCl_3$)

**Beispiel 5**

(S)-4-(5-Octyloxy-pyrimidin-2-yl)phenyl-(2,2-dimethyl-1,3-dioxolan-4-yl)methyl-ether

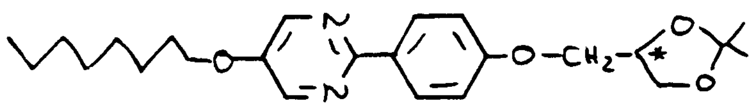

Schmelzpunkt: 94,2 °C $[\alpha[^{22}_{D}$ : + 8,3 (c = 5, $CHCl_3$)

**Beispiel 6**

(S)-4-[5-(4-Hexyl-phenyl)-pyrimidin-2-yl]phenyl-(2,2-dimethyl-1,3-dioxolan-4-yl)methyl-ether

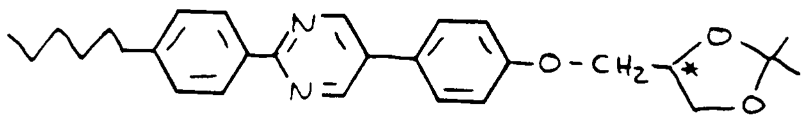

Schmelzpunkt: 110 °C $[\alpha]^{22}_{D}$ : + 3,9 (c = 5, $CHCl_3$) Klärpunkt: 164 °C

**Beispiel 7**

(S)-4-(4-Decyloxy-benzoyloxy)phenyl-(2,2-dimethyl-1,3-dioxolan-4-yl)methyl-ether

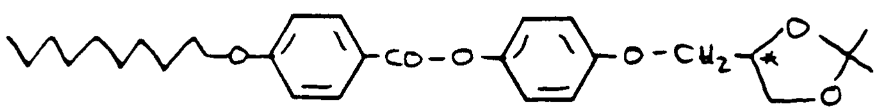

Schmelzpunkt: 70,4 °C $[\alpha]^{22}_{D}$ : + 4,9 (c = 5, $CHCl_3$)

**Beispiel 8**

(S)-[4-(4-Octyloxy-benzoyloxy)benzoyloxy]phenyl-(2,2-dimethyl-1,3-dioxolan-4-yl)methyl-ether

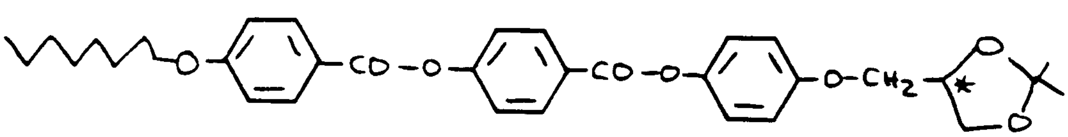

X 126 $S_A^*$ 149 N* 175 I $\alpha]^{22}_{D}$ : + 3,6 (c = 5, $CHCl_3$)
Diese Verbindung ist selbst flüssigkristallin und kann daher - neben der Funktion als chiraler Dotierstoff - auch zur Erweiterung des $S_A^*$ und N*-Phasenbereichs von flüssigkristallinen Mischungen eingesetzt werden.

**Beispiel 9**

(S)-4-[5-(1H,1H-Perfluoroctyloxy)-pyrimidin-2-yl]phenyl-(2,2-dimethyl-1,3-dioxolan-4-yl)methyl-ether

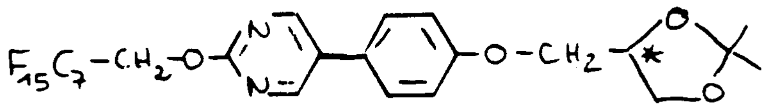

Schmelzpunkt: 98,9 °C $[\alpha]_D^{22}$ : + 4,0 (c = 5, $CHCl_3$)

**Beispiel 10**

(S)-4-[5-(7-Methyl-nonyloxy)pyrimidin-2-yl]phenyl-(2,2-dimethyl-1,3-dioxolan-4-yl)methyl-ether

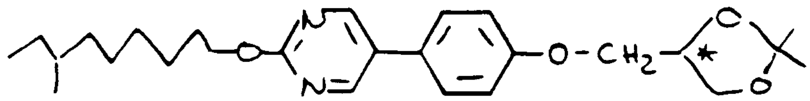

X 56 $S_A$ 57 I $[\alpha]_D^{22}$ : + 9,7 (c = 6, $CHCl_3$)
Das Auftreten einer $S_A$-Phase führt zu einer besonders guten Verträglichkeit dieser Verbindung in smektischen Phasen.

Analog, jedoch unter Verwendung von (S)-2-Oxo-1,3-dioxolan-4-methanol bzw. dem 5-Propyl-4-methanol-Derivat, werden erhalten:

**Beispiel 11**

(S)-4-(5-Octyl-pyrimidin-2-yl)phenyl-(2-oxo-1,3-dioxolan-4-yl)methyl-ether

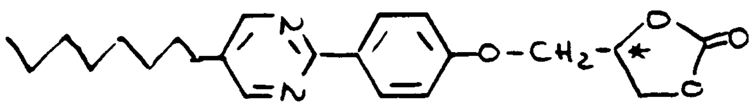

Schmelzpunkt: 122,4 °C $[\alpha]_D^{22}$ : - 5,3 (c = 5, $CHCl_3$)

**Beispiel 12**

(S)-4-(2-Octylthio-pyrimidin-5-yl)phenyl-(2-oxo-1,3-dioxolan-4-yl)methyl-ether

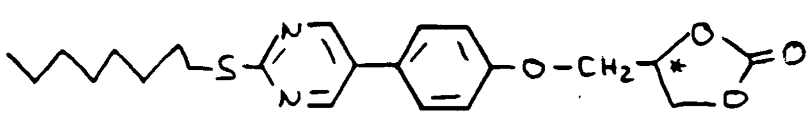

Schmelzpunkt: 104,9 °C $[\alpha]_D^{22}$ : - 10,0 (c = 5, $CHCl_3$)

**Beispiel 13**

(S)-4-(2-Octyloxy-pyrimidin-5-yl)phenyl-(2-oxo-1,3-dioxolan-4-yl)methyl-ether

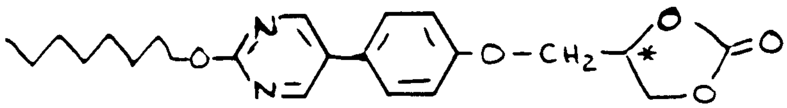

Schmelzpunkt: 117,8 °C $[\alpha]_D^{22}$ : - 8,9 (c = 5, $CHCl_3$)

**Beispiel 14**

4-Dodecyloxy-biphenyl-4'-yl-(2-oxo-5-propyl-1,3-dioxolan-4-yl)methyl-ether

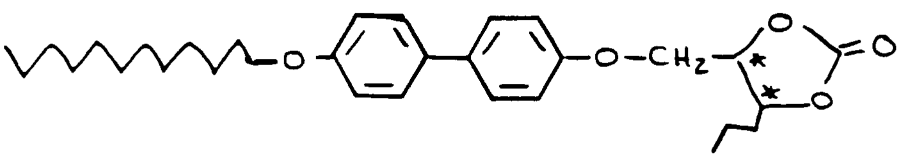

Schmelzpunkt: 94 °C

**Beispiel 15**

cis-4-(4-Dodecyloxy-benzoyloxy)-biphenyl-4'-yl-(2-oxo-5-propyl-1,3-dioxolan-4-yl)methyl-ether

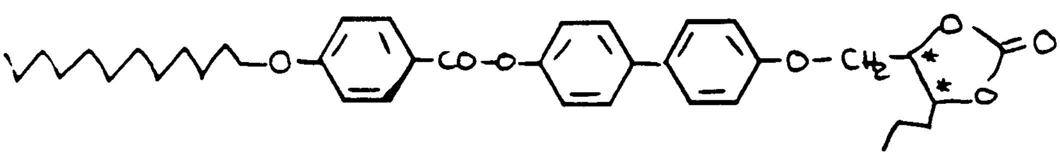

Schmelzpunkt: 122,5 °C

**Beispiel 16**

trans- 4-(4 -Decyloxy-benzoyloxy)-biphenyl-4'-yl-(2-oxo-5-propyl-1,3-dioxolan-4-yl)methyl-ether

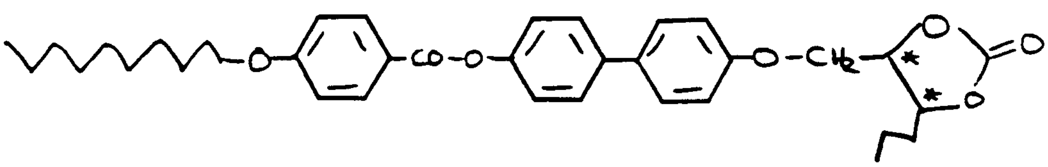

Phasenfolge: K 133,5 $S_A$ 146,5 I

**Beispiel 17**

cis-[4-(5-Octyloxy-pyrimidin-2-yl)phenyl]-(2-oxo-5-propyl-1,3-dioxolan-4-yl)methyl-ether

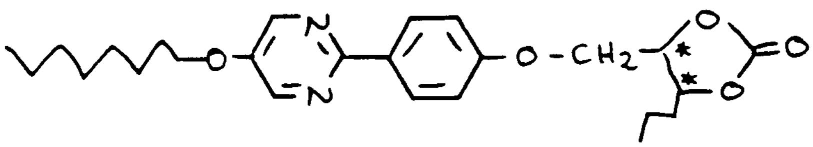

Schmelzpunkt: 100 °C

**Beispiel 18**

trans-[4-(5-Octyloxy-pyrimidin-2-yl)phenyl]-(2-oxo-5-propyl-1,3-dioxolan-4-yl)methyl-ether

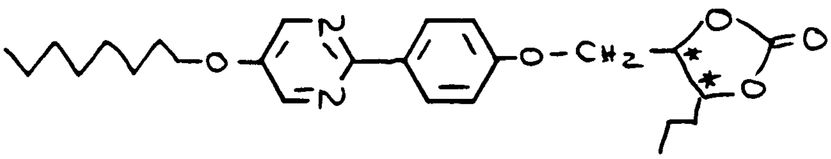

Schmelzpunkt: 83 °C

**Beispiel 19**

(S)-4-(2-Octyloxy-pyrimidin-5-yl)phenyl-[spiro(1,3-dioxolan-2,1′-cyclohexan)-4-yl]methyl-ether

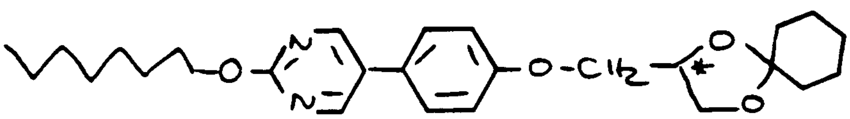

Eine Lösung von 3 g 4-(2-Octyloxy-pyrimidin-5-yl)phenol in 100 ml Dimethylformamid wird mit 0,72 g NaH (50 %ig in Paraffinöl) versetzt und nach Abklingen der Reaktion 4,89 g (S)-2,3-O-Cyclohexylidenglycerin-tosylat zugegeben. Nach 4 h wird mit 500 ml $H_2O$ versetzt, mit Dichlormethan die wäßrige Mischung extrahiert und der Extrakt chromatographisch aufgetrennt ($SiO_2$, $CH_2Cl_2$/Essigsäureethylester 99/1). Nach dem Umkristallisieren aus n-Hexan werden 2,0 g des gewünschten Produkts vom Schmelzpunkt 93,4 °C erhalten.
$[\alpha]_D^{22}$ : + 8,0 (c = 5, $CHCl_3$)
Analog werden erhalten:

**Beispiel 20**

(S)-4-(2-Octylthio-pyrimidin-5-yl)phenyl-[spiro(1,3-dioxolan-2,1′-cyclohexan)-4-yl]methyl-ether

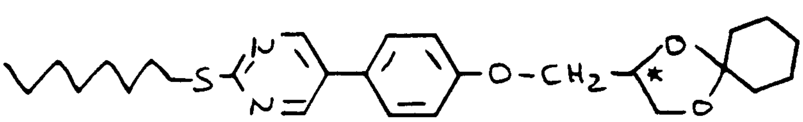

Schmelzpunkt: 80,2 °C $[\alpha]_D^{22}$ : + 8,6 (c = 5, $CHCl_3$)

**Beispiel 21**

(S)-4-(2-Octyl-pyrimidin-5-yl)phenyl-[spiro(1,3-dioxolan-2,1'-cyclohexan)-4-yl]methyl-ether

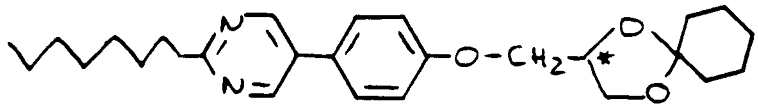

Schmelzpunkt: 91,3 °C $[\alpha]_D^{22}$ : + 8,3 (c = 5, $CHCl_3$)

**Beispiel 22**

(S)-4[-<4-(5-Octyl-pyrimidin-2-yl)phenyloxy>-carbonyloxymethyl]-2,2-dimethyl-1,3-dioxolan

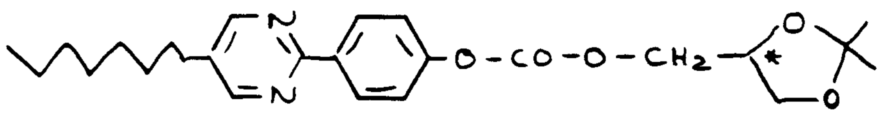

Eine Lösung von 4,3 g 4-(5-Octyl-pyrimidin-2-yl)phenol und 2,1 g N,N-Dimethylanilin in 50 ml Toluol wird mit 9,9 g einer 15 Gew.-%igen Lösung von Phosgen in Toluol versetzt. Nach 24 h wird filtriert und das Filtrat mit 1,2 g Pyridin und tropfenweise bei 0 °C mit 2 g (S)-2,2-Dimethyl-1,3-dioxolan-4-methanolvermischt. Nach 6 h wird filtriert, im Vakuum das Filtrat vom Lösemittel befreit und der Rückstand chromatographisch aufgetrennt ($SiO_2$, $CH_2Cl_2$). Nach dem Umkristallisieren aus n-Hexan werden 1,4 g des gewünschten Produkts vom Schmelzpunkt 47 °C erhalten.
$[\alpha]^{22}$: - 5,1 (c = 1,5, $CHCl_3$)

**Meßmethode:**

Versetzt man ein (nicht-chirales) Lösemittel mit einer kleinen Menge einer chiralen Verbindung, so wird die Ebene des linear polarisierten Lichts um den (charakteristischen) Winkel $\alpha$ gedreht; dieser Winkel wird wie folgt angegeben: $[\alpha]_D^T$ (c = x, LM), wobei die Symbole folgende Bedeutung haben: x = Konzentration der Lösung in g/l, LM = Lösemittel, D = 589 nm (NaD-Linie), T = Temperatur der Lösung. Der Drehwinkel wird in einem Polarimeter nach 10 cm Durchgang des Lichts bestimmt.

**Anwendungsbeispiele A1 bis A13**

Die Bestimmung der HTP ("helical twisting power")-Werte, d. h. einem Maß für das Verdrillungsvermögen, wird nach dem bei Kassubek et al., Mol. Cryst. Liq. Cryst. 8, 305-314 (1969) beschriebenen Verfahren durchgeführt. Dazu werden die chiralen Dotierstoffe in eine nematische Wirtsmischung eingemischt; mit dieser Testmischung wird eine planar-orientierende Keilzelle gefüllt und durch Ausmessen der Versetzungslinien im Polarisationsmikroskop (bei bekanntem Keilwinkel der Zelle) die cholesterische Helixganghöhe bestimmt. Das Vorzeichen der Ganghöhe kann durch Drehen des Analysators bestimmt werden. Ist die Ganghöhe (oder "pitch") p bekannt, so berechnet man die HTP nach: HTP = 1p•X mit X = Molenbruch des chiralen Dotierstoffes. Die eingesetzten Wirtsmischungen werden mit A bis D bezeichnet. Die HTP-Werte sind der Tabelle 1 zu entnehmen. Die Wirtsmischungen besitzen folgende Bereiche der cholesterischen Phase:

| | |
|---|---|
| A | 78 bis 83 °C |
| B | 77 bis 98 °C |
| C | 90 bis 105 °C |
| D | 61 bis 67 °C |

Die Ergebnisse zeigen, daß große HTP-Werte erhältlich sind und damit ein weiter Bereich an gewünschten Ganghöhen durch geringe Zugabemengen der erfindungsgemäßen Verbindungen ermöglicht wird.

Tabelle 1

| Anw.- Beispiele | Dotierstoff aus Bsp. | Wirt | Molenbruch | Temp. (°C) | p (µm) | HTP ($\mu m^{-1}$) |
|---|---|---|---|---|---|---|
| A 1 | 1 | A | 0,05 | 78 | - 2,6 | -7,7 |
| A 2 | 2 | B | 0,02 | 91 | - 5,9 | -8,5 |
| A 3 | 2 | C | 0,02 | 91 | - 8,5 | -5,9 |
| A 4 | 5 | D | 0,044 | 66 | - 3,2 | -7,1 |
| A 5 | 11 | B | 0,02 | 83 | -42 | -1,2 |
| A 6 | 19 | B | 0,02 | 90 | - 6,7 | -7,5 |
| A 7 | 22 | B | 0,02 | 94 | -45 | -1,1 |

Für den Einsatz von Dotierstoffen zur pitch-Kompensation ferroelektrischer (smektisch C*) Flüssigkristallmischungen ist es vorteilhaft, wenn zwar das Verdrillungsvermögen groß, aber die gleichzeitig induzierte spontane Polarisation $P_s$ (in der smektisch C*-Phase) möglichst gering ist, so daß dann nicht oder kaum auf die Vorzeichenkompatibilität mit weiteren chiralen Mischungskomponenten geachtet werden muß. Mit dem Einsatz der erfindungsgemäßen Verbindungen ist dies möglich (siehe Tabelle 2). Die $P_s$-Werte werden nach der Sawyer-Tower-Methode [siehe K. Skarp et al., Ferroelectric Letters 6, 67 (1986)] bei 25 °C in 10 Gew.-%igen Testmischungen der Dotierstoffe in der Wirtsmischung D (smektische C*-Phase von 14 bis 51 °C) bestimmt; praktisch verwendbare Mischungen sollten $P_s$-Werte von ≧ 5 nC/cm² haben, d. h. die erwünschte Zielsetzung einer niedrigen spontanen Polarisation ist erreicht.

Tabelle 2

| Anw.- Beispiele | Dotierstoff aus Bsp. | spontane Polarisation $P_s$ (nC/cm²) |
|---|---|---|
| A 8 | 1 | 0,4 |
| A 9 | 3 | < 0,2 |
| A 10 | 4 | 0,5 |
| A 11 | 5 | 0,3 |
| A 12 | 11 | < 0,2 |
| A 13 | 22 | 2 |

## Patentansprüche

**1.** Optisch aktive, in 4-Stellung einen mesogenen Rest tragende 1,3-Dioxolan-Derivate der allgemeinen Formel (I)

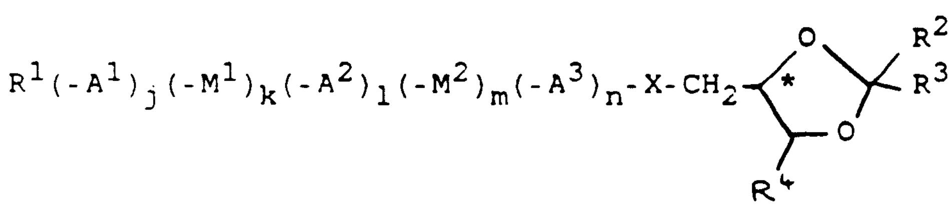

(I)

in der die Symbole folgende Bedeutung haben:

$R^1$

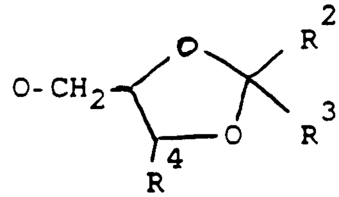

oder
ein geradkettiger oder verzweigter Alkylrest mit 1 bis 16 C-Atomen oder ein geradkettiger oder verzweigter Alkenylrest mit 3 bis 16 C-Atomen, wobei diese Reste selbst asymmetrische C-Atome enthalten können, wobei eine oder mehrere nicht benachbarte $-CH_2$-Gruppen durch -O-, -S-, -CO-, -O-CO- und/oder -CO-O- und wobei ein oder mehrere H durch F, Cl, Br oder CN ersetzt sein können

$R^2$ und $R^3$ jeweils H oder ein Alkylrest mit 1 bis 10 C-Atomen, wobei ein oder mehrere H durch F ersetzt sein können, oder $R^2$ und $R^3$ bilden zusammen mit dem C(2)-Atom des Dioxolanrings einen Cyclopentan-, Cyclohexan- oder Cycloheptanring oder eine Ketogruppe

$R^4$ H oder ein Alkylrest mit 1 bis 10 oder ein Alkenylrest mit 2 bis 10 C-Atomen

j und l null, 1 oder 2

k und m null oder 1

n null, 1 oder 2

mit folgender Maßgabe: wenn j und/oder l = null sind, ist k = null; wenn n = null ist, ist m = null; die Summe j + l + n ist minimal 1 und maximal 3,

$-A^1$ und $-A^2$

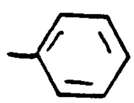 , 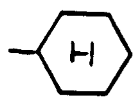 , 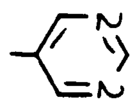 , 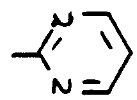 ,

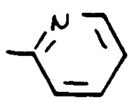 , 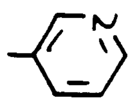 , 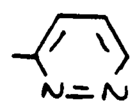 , 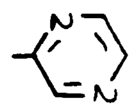 ,

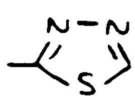 , 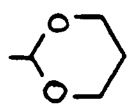 , 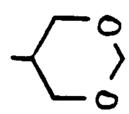 , 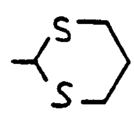 ,

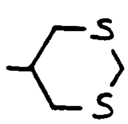 , 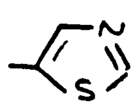 , 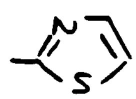 ,

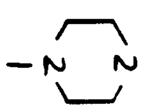 , 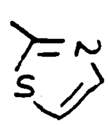 , 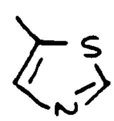

$-A^3$

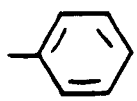 , 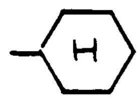 , 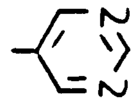 , 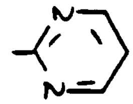 ,

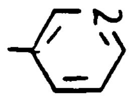 , 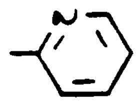 , 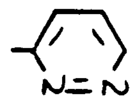

| | |
|---|---|
| $-M^1$ und $-M^2$ | -CO-O, -O-CO, $-CH_2CH_2$, -CH=CH, $-CH_2O$, $-OCH_2$, -C≡C |
| X | O, S oder O-CO-O. |

**2.** 1,3-Dioxolan-Derivate nach Anspruch 1 der allgemeinen Formel (IV)

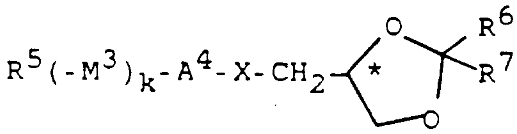 (IV)

worin bedeuten:

| | |
|---|---|
| $R^6$, $R^7$ | Methyl oder zusammen mit dem C(2)-Atom des Dioxolanrings einen Cyclohexanring |
| $R^5$ | einen geradkettiger oder verzweigter Alkyl- oder Alkenylrest mit 6 bis 12 C-Atomen, der ein asymmetrisches C-Atom enthalten kann, |
| $-M^3$ | -O, -S oder -O-CO, |
| $-A^4$ | |

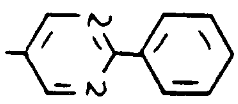 , 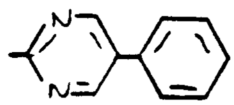 , 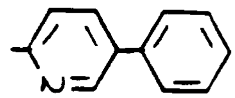 ,

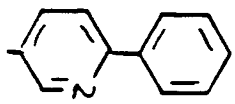 , 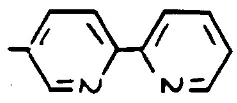 , 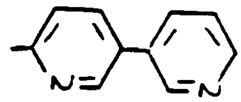 ,

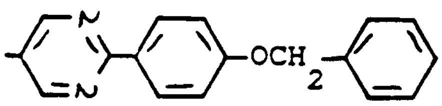 , 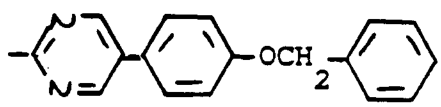 ,

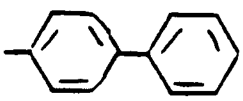 , 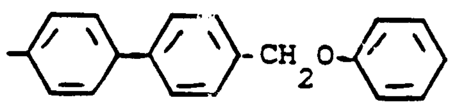 ,

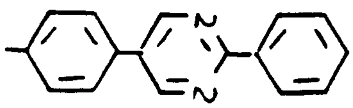 , 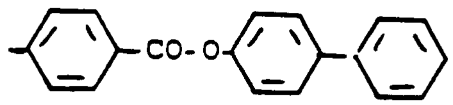 ,

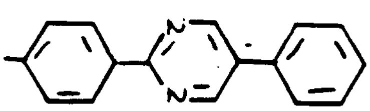 oder 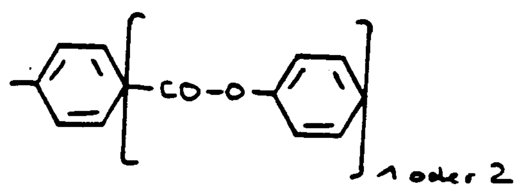

**3.** Flüssigkristallmischung, gekennzeichnet durch einen Gehalt an mindestens einem optisch aktiven 1,3-Dioxolan-Derivat der allgemeinen Formel (I) nach Anspruch 1.

**4.** Ferroelektrische Flüssigkristallmischung, gekennzeichnet durch einen Gehalt an mindestens einem optisch aktiven 1,3-Dioxolan-Derivat der allgemeinen Formel (I) nach Anspruch 1.

**5.** Flüssigkristallmischung, gekennzeichnet durch einen Gehalt an mindestens einem optisch aktiven und mesogenen 1,3-Dioxolan-4-Derivat der allgemeinen Formel (IV) nach Anspruch 2.

**6.** Elektrooptisches Schalt- oder Anzeigeelement enthaltend eine Flüssigkristallmischung nach einem der Ansprüche 3 bis 5.

**7.** Verfahren zur Herstellung eines optisch aktiven 1,3-Dioxolan-Derivates der allgemeinen Formel (I) nach Anspruch 1, dadurch gekennzeichnet, daß mesogene Phenole, Thiophenole oder Kohlensäuremonoester der allgemeinen Formel (II)

$R^1(-A^1)_j(-M^1)_k(-A^2)_l(-M^2)_m(-A^3)_n\text{-X-H}$ (II)

mit geeigneten Derivaten des 1,3-Dioxolans (III)

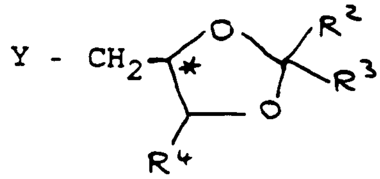 (III)

umgesetzt werden, wobei Y eine geeignete Abgangsgruppe ist.

## Claims

**1.** An optically active 1,3-dioxolane derivative which carries a mesogenic radical in the 4-position and has the formula (I)

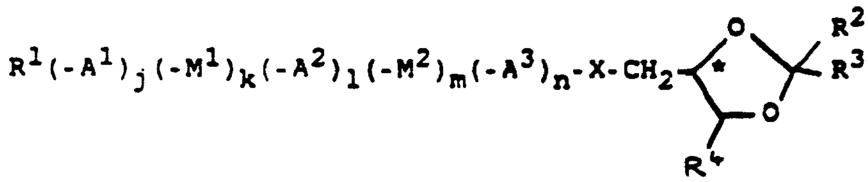 (I)

in which the symbols have the following meanings:

$R^1$ is

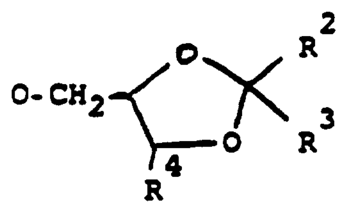

or

a linear or branched alkyl radical having 1 to 16 carbon atoms or a linear or branched alkenyl radical having 3 to 16 carbon atoms, it being possible for these radicals themselves to contain asymmetric carbon atoms and for one or more non-adjacent $-CH_2-$ groups to be replaced by -O-, -S-, -CO-, -O-CO- and/or -CO-O-, and for one or more H atoms to be replaced by F, Cl, Br or CN,

$R^2$ and $R^3$ are each H or an alkyl radical having 1 to 10 carbon atoms, it being possible for one or more H atoms to be replaced by F, or $R^2$ and $R^3$, together with the C(2) atom of the dioxolane ring, form a cyclopentane, cyclohexane or cycloheptane ring or a keto group,

$R^4$ is H or an alkyl radical having 1 to 10 carbon atoms or an alkenyl radical having 2 to 10 carbon atoms,

j and 1 are zero, 1 or 2,

k and m are zero or 1,

n is zero, 1 or 2, subject to the following proviso: if j and/or l are zero, k is zero; if n is zero, m is zero; the sum of j + l + n is not less than 1 and not more than 3,

$-A^1$ and $-A^2$ are

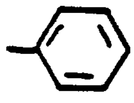 , 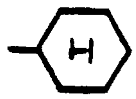 , 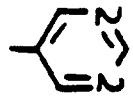 , 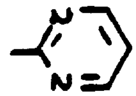 ,

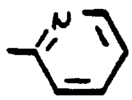 , 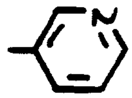 , 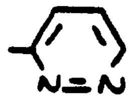 , 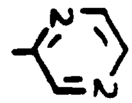 ,

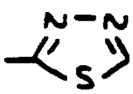 , 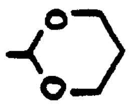 , 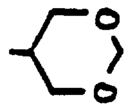 , 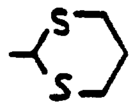 ,

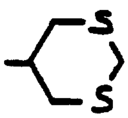 , 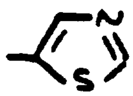 , 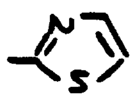 ,

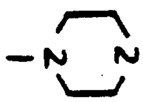 , 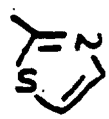 , 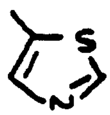

-A$^3$

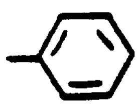 , 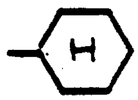 , 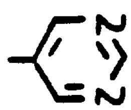 , 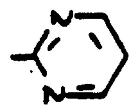 ,

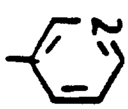 , 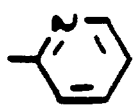 , 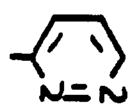

| | |
|---|---|
| -M$^1$ and -M$^2$ | are -CO-O, -O-CO, $-CH_2CH_2$, -CH = CH, $-CH_2O$, $-OCH_2$ or -C≡C and |
| X is | O, S or O-CO-O. |

**2.** A 1,3-dioxolane derivative as claimed in claim 1, of the formula (IV)

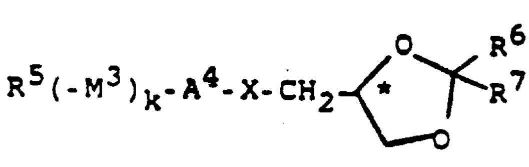 (IV)

in which

| | |
|---|---|
| $R^6$ and $R^7$ | denote methyl or, together with the C(2) atom of the dioxolane ring, denote a cyclohexane ring, |
| $R^5$ | denotes a linear or branched alkyl or alkenyl radical which has 6 to 12 carbon atoms and which can contain an asymmetric carbon atom, |
| $-M^3$ | denotes -O, -S or -O-CO and |
| $-A^4$ | denotes |

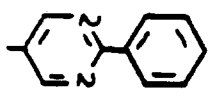 , 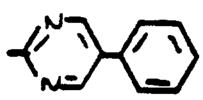 , 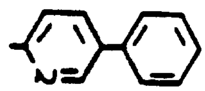

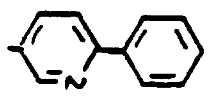 , 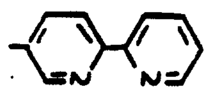 , 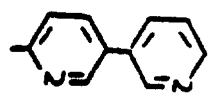 ,

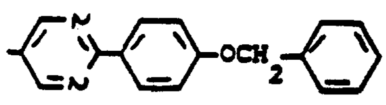 , 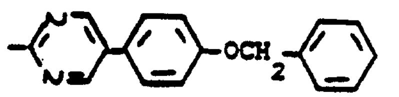 ,

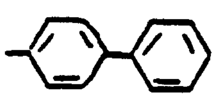 , 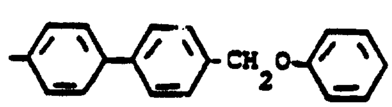 ,

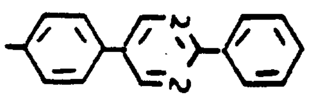 , 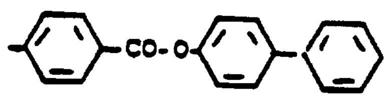 ,

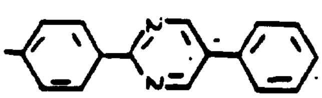 or 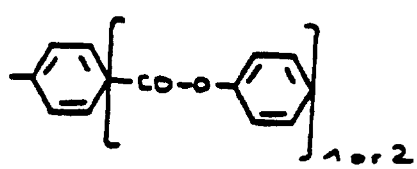

3. A liquid-crystal mixture which contains at least one optically active 1,3-dioxolane derivative of the formula (I) as claimed in claim 1.

4. A ferroelectric liquid-crystal mixture which contains at least one optically active 1,3-dioxolane derivative of the formula (I) as claimed in claim 1.

5. A liquid-crystal mixture which contains at least one optically active and mesogenic 1,3-dioxolan-4-yl derivative of the formula (IV) as claimed in claim 2.

6. An electrooptical switching element or display element containing a liquid-crystal mixture as claimed in one of claims 3 to 5.

7. A process for the preparation of an optically active 1,3-dioxolane derivative of the formula (I) as claimed in claim 1, which comprises reacting mesogenic phenols, thiophenols or carbonic acid monoesters of

the formula (II)

$R^1(-A^1)_j(-M^1)_k(-A^2)_l(-M^2)_m(-A^3)_n$-X-H (II)

with suitable derivatives of the 1,3-dioxolane (III)

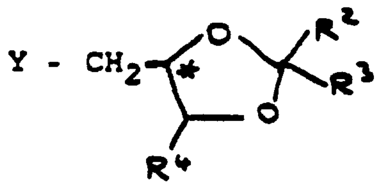

(III)

in which Y is a suitable leaving group.

**Revendications**

**1.** Dérivés optiquement actifs du dioxolanne-1,3 portant un reste mésogène en position 4, ces dérivés ayant la formule générale (I)

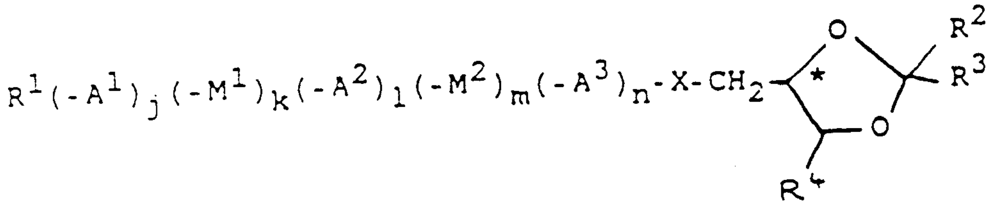

(I)

dans laquelle les symboles ont la signification suivante :
$R^1$ représente

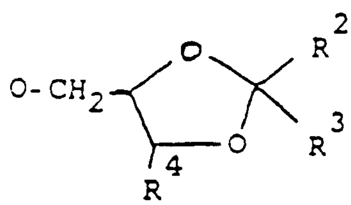

ou
un reste alkyle à chaîne droite ou ramifiée ayant de 1 à 16 atomes de carbone ou un reste alcényle à chaîne droite ou ramifiée ayant de 3 à 16 atomes de carbone, ces restes pouvant même contenir des atomes de carbone asymétrique, un ou plusieurs groupes $-CH_2$ - non voisins pouvant être substitués par -O-, -S-, -CO-, -O-CO-, et/ou -CO-O- et un ou plusieurs H pouvant être substitués par F, Cl, Br ou CN,
$R^2$ et $R^3$ représentent chacun H ou un reste alkyle ayant de 1 à 10 atomes de C, un ou plusieurs H pouvant être substitués par F, ou $R^2$ et $R^3$ forment ensemble avec l'atome C(2) du cycle du dioxolanne un cycle cyclopentane, cyclohexane ou cycloheptane ou un groupe céto;
$R^4$ représente H ou un reste alkyle ayant de 1 à 10 atomes de C ou un reste alcényle ayant de 2 à 10 atomes de C,
j et l sont 0, 1 ou 2,
k et m sont 0 ou 1,
n est 0, 1 ou 2,
avec les conditions suivantes : si j et/ou l = 0, k = 0; si n = 0, m = 0; la somme j + l + n est au moins 1 et au plus 3,

$-A^1$ et $-A^2$ représentent

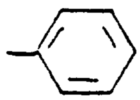 , 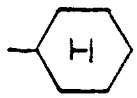 , 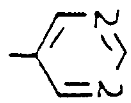 , 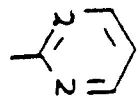 ,

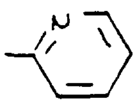 , 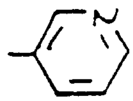 , 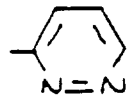 , 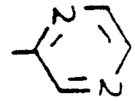 ,

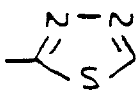 , 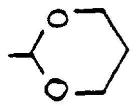 , 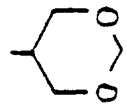 , 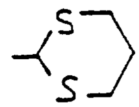 ,

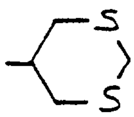 , 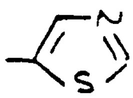 , 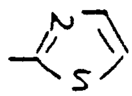 ,

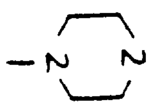 , 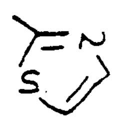 , 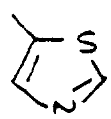

$-A^3$ représente

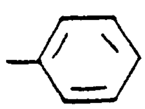 , 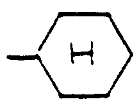 , 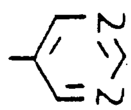 , 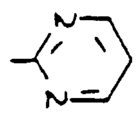 ,

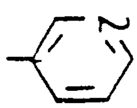 , 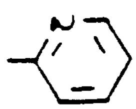 , 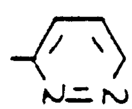

$-M^1$ et $-M^2$ représentent
-CO-O, -O-CO, $-CH_2CH_2$, -CH = CH, $-CH_2O$, $-OCH_2$ -C≡C
X représente O, S ou O-CO-O.

2. Dérivés du dioxolanne-1,3 selon la revendication 1, de formule générale (IV)

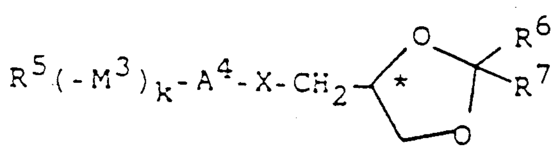
(IV)

dans laquelle :
$R^6$, $R^7$ représentent le méthyle, ou, avec l'atome C(2) du cycle du dioxolanne, un cycle du cyclohexane,
$R^5$ représente un reste alkyle ou alcényle à chaîne droite ou ramifiée ayant de 6 à 12 atomes de C, qui peut comporter un atome de C asymétrique,
$M^3$ représente -O, -S ou -O-CO,
-$A^4$ représente

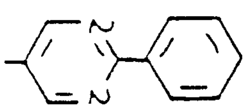 , 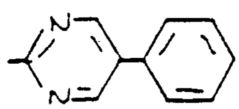 , 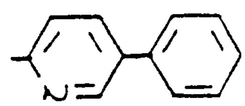 ,

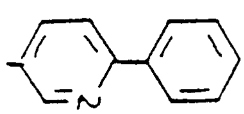 , 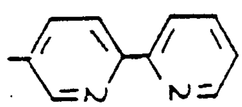 , 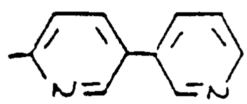 ,

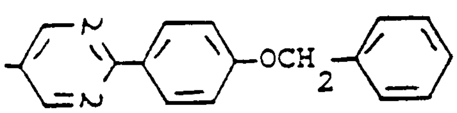 , 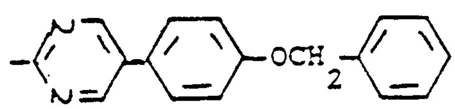 ,

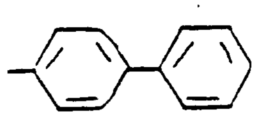 , 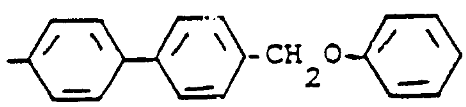 ,

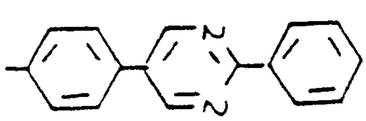 , 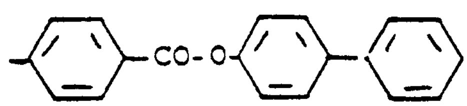 ,

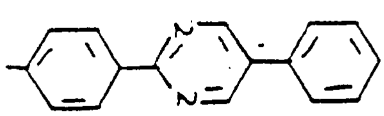 ou 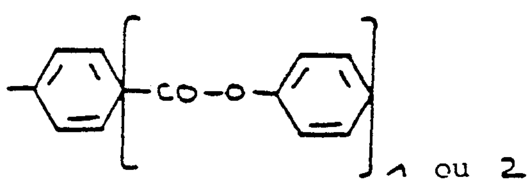

3. Mélange de cristaux liquides, caractérisé en ce qu'il contient au moins un dérivé optiquement actif du dioxolanne-1,3 de formule générale (I) selon la revendication 1.

**4.** Mélange de cristaux liquides ferroélectrique, caractérisé en ce qu'il contient au moins un dérivé optiquement actif du dioxolanne-1,3 de formule générale (I) selon la revendication 1.

**5.** Mélange de cristaux liquides, caractérisé en ce qu'il contient au moins un dérivé -4 optiquement actif et mésogène du dioxolanne-1,3 de formule générale (IV) selon la revendication 2.

**6.** Elément électro-optique de commutation ou d'affichage comportant un mélange de cristaux liquides selon l'une des revendications 3 à 5.

**7.** Procédé pour préparer un dérivé optiquement actif du dioxolanne-1,3 de formule générale (I) selon la revendication 1, caractérisé en ce que l'on fait réagir des phénols, des thiophénols ou des monoesters d'acides carboxyliques, mésogènes, de formule générale (II)

$R^1(-A^1)_j(-M^1)_k(-A^2)_l(-M^2)_m(-A^3)_n$-X-H (II)

avec des dérivés appropriés du dioxolanne-1,3 (III)

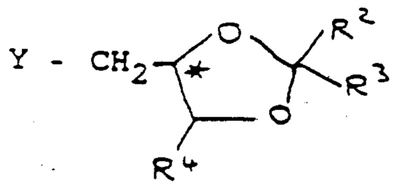

(III)

où Y est un groupe de départ approprié.